# EUROPÄISCHE PATENTSCHRIFT

(11) **EP 1 530 944 B1**
(45) Veröffentlichungstag und Bekanntmachung des Hinweises auf die Patenterteilung: **05.03.2008**
(21) Anmeldenummer: 04019338.5
(22) Anmeldetag: 14.08.2004
(51) Int. Cl.: A61B 1/227, A61B 1/06

(54) **Medizinisches Diagnostikgerät**
Medical diagnostic apparatus
Appareil de diagnostique médical

(30) Priorität: 15.11.2003 DE 20317671 U
(43) Veröffentlichungstag der Anmeldung: 18.05.2005
(73) Patentinhaber: Kirchner & Wilhelm GmbH + Co. KG, 71679 Asperg (DE)
(72) Erfinder: Kirchner, Regina, 71706 Markgröningen (DE)
(74) Vertreter: Kohler Schmid Möbus

(56) Entgegenhaltungen:
- WO-A-02/071930
- WO-A-03/047415
- WO-A-20/04084716
- DE-U- 20 219 984
- GB-A- 2 374 402
- US-A1- 2002 038 075

## Beschreibung

Die Erfindung bezieht sich auf ein medizinisches Diagnostikgerät mit Beleuchtung, wobei das Gerät aus einem, die Batterien aufnehmenden Handgriff oder dergleichen und einem auf diesem aufgesetzten Kopf besteht. Es ist bereits bekannt, zur Beleuchtung statt der bisher üblichen herkömmlichen Glühlampen Leuchtdioden zu verwenden, da die Glühlampen einen relativ hohen Strombedarf haben und auch störende Wärme bei der Untersuchung abstrahlen. Leuchtdioden geben dagegen eine kaum spürbare Wärme ab, nehmen relativ wenig Strom auf und haben eine außerordentlich lange Lebensdauer. Die Leuchtdioden benötigen allerdings einen Schaltwandler, um von den handelsüblichen Batterien gespeist werden zu können. Bei bekannten Ausführungsformen ist dabei der Schaltwandler im Kopf bzw. im Halsteil des Gerätes angeordnet.

Aus der GB--2 374 402 ist bereits ein solches Diagnostikgerät mit Beleuchtung durch eine, über eine Batterie und einen Schaltwandler gespeiste Leuchtdiode bekannt. Dabei sind die Batterie und der, als Hülse gestaltete Schaltwandler in einem Handgriff aufgenommen, auf welchem ein Kopfteil aufgesetzt ist. Dabei erfolgen die elektrischen Verbindungen mit der Batterie und der Leuchtdiode über die Hülse. Eine ähnliche Ausführungsform zeigt auch die ältere Anmeldung WO 2004/084716 A (Dokument nach Artikel 54(3) EPÜ), in der die Hülse als Batterie gestaltet ist.

Um den Aufbau eines solchen Gerätes zu vereinfachen weist erfindungsgemäß die Hülse die Form einer handelsüblichen Batterie auf und weist außen einen aus Metall bestehenden Mantel auf, ist oben durch eine Kunststoffbuchse abgeschlossen und nimmt unten einen Kunststoffsockel auf, auf welchem sich der Schaltwandler abstützt.

Vorzugsweise ist die Kunststoffbuchse mit einem Durchtritt für Stromleiter versehen. Ferner ist es zweckmässig, dass die Hülse an ihrem unteren Ende über eine Kontaktfläche mit dem einen Pol und seitlich über eine innerhalb des Handgriffes verlaufende Leitung mit dem anderen Pol der Batterie verbunden ist.

Der Handgriff kann nunmehr für alle Arten von Diagnostikgeräten mit den verschiedensten Lichtquellen und in unterschiedlichen Größen verwendet werden.

Die Handgriffe sind üblicherweise für die Aufnahme von mindestens zwei Batterien ausgebildet. Man kann daher den gleichen Handgriff sowohl mit zwei oder mehreren Batterien für Geräte mit herkömmlichen Glühlampen als auch mit einer Hülse mit Schaltwandler und einer Batterie weniger für Leuchtdioden verwenden. Da, wie bereits oben erwähnt, Leuchtdioden wesentlich weniger Strom als eine Glühlampe verbrauchen, kann bei der Anwendung von Leuchtdioden durchaus auf eine Batterie verzichtet werden. Ein weiterer Vorteil der Hülse ist es, dass sie auch durch den Benutzer leicht ausgewechselt werden kann und ein falscher Einbau praktisch ausgeschlossen ist. Vor allem von Bedeutung ist es, dass auch die bisher üblichen Handgriffe nach wie vor ohne jede Änderung weiterverwendet werden können.

Die Leuchtdiode oder die Leuchtdioden können im Kopf angeordnet und über stromführende Leitungen mit dem Schaltwandler in der Hülse verbunden sein. Bei einem anderen Ausführungsbeispiel der Erfindung kann die Hülse außer dem Schaltwandler ein oder mehrere Leuchtdioden an ihrem zum Kopf gerichteten Ende aufweisen, wobei dann der Lichtstrahl über ein oder mehrere Lichtleiter dem Kopf zugeführt wird. Dabei ist es zweckmässig die Leuchtdiode bzw. die Leuchtdioden in der Hülse anzuordnen, wobei sie durch ein oder mehrere Ausnehmungen derselben hindurchragen.

Die Zeichnung zeigt ein Ausführungsbeispiel der Erfindung. Es stellen dar:
- Figur 1: die Ansicht eines Otoskops, wobei der Kopf mit Hals vom Handgriff getrennt ist,
- Figur 2: einen Querschnitt nach Fig. 1,
- Figur 3: einen Querschnitt nach Fig. 2 einer anderen Ausführungsform,
- Figur 4: einen Querschnitt durch eine Hülse nach Fig. 2 mit Schaltwandler in vergrößertem Maßstab,
- Figur 5: einen Querschnitt nach Fig. 4 einer Ausführungsform nach Fig. 3.

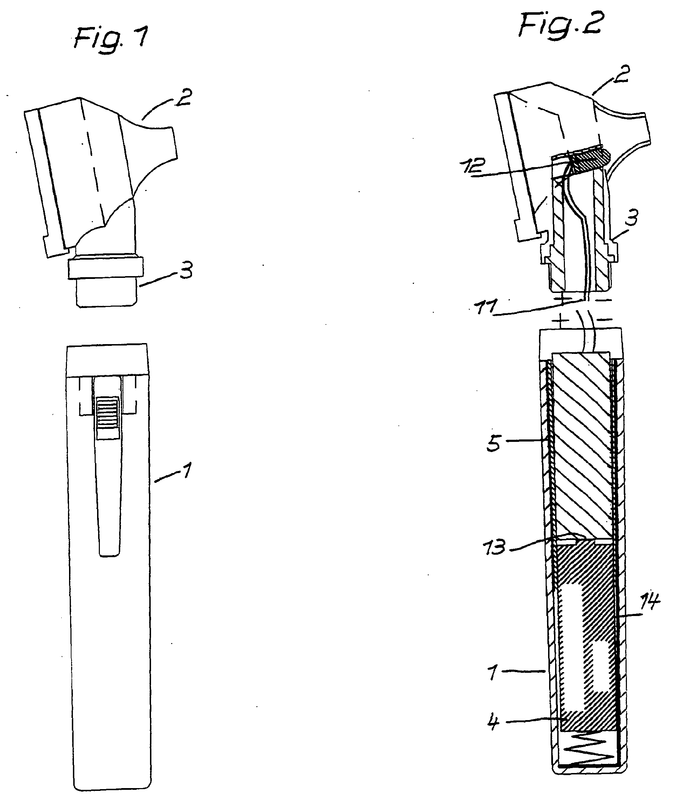

Das in den Fign. 1 und 2 dargestellte Otoskop besteht aus einem Handgriff 1 zur Aufnahme von Batterien und einem Kopf 2 mit Hals 3, welcher auf den Handgriff 1 aufsteckbar ist. Im Ausführungsbeispiel nach Fig. 2 nimmt der Handgriff 1 im unteren Teil eine Batterie 4 auf, während im oberen Teil statt einer zweiten Batterie eine den Abmessungen einer Batterie angepasste Hülse 5 angeordnet ist.

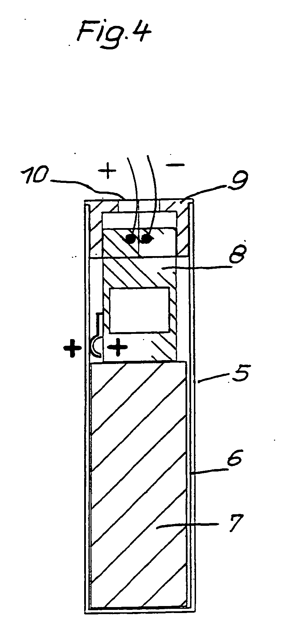

Die in Fig. 4 dargestellte Hülse 5 weist einen äußeren, aus Metall bestehenden Mantel 6 auf, welcher im unteren Teil einen Kunststoffsockel 7 aufnimmt. Auf diesem stützt sich ein Schaltwandler 8 ab, welcher die von der Batterie 4 zugeführte Spannung an die für eine Leuchtdiode erforderliche anpasst. Im oberen Teil ist die Hülse 5 durch einen Kunststoffstopfen 9 abgeschlossen. Dieser weist eine Ausnehmung 10 zum Durchtritt von elektrischen Leitungen 11 auf, welche zur Leuchtdiode 12 (Fig. 2) im Kopf 2 führen. Der Schaltwandler 8 wird von der Batterie 4 durch Anliegen des Pluspols 13 derselben am Metallmantel 6 und vom Minuspol über den Leiter 14 gespeist.

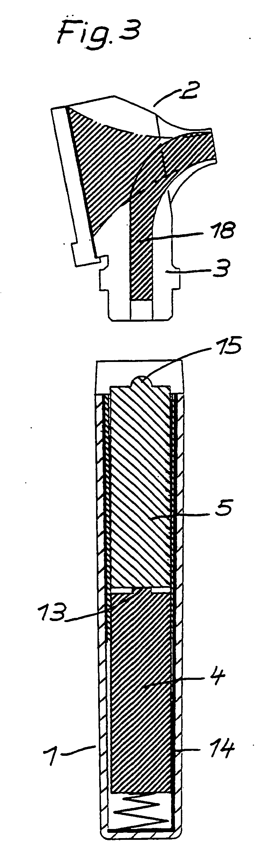

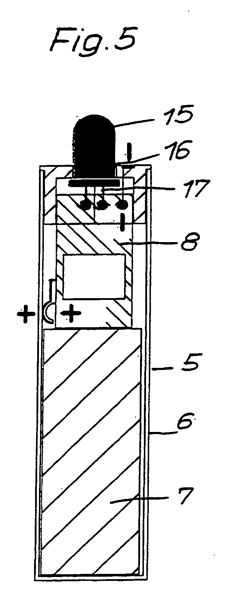

Im Ausführungsbeispiel nach Fig. 3 ist die Leuchtdiode 15 nicht im Kopf, sondern wie Fig. 5 zeigt, unmittelbar an der Hülse 5 angeordnet. Dabei weist der Kunststoffstopfen 9 eine größere Ausnehmung 16 auf, durch welche die Leuchtdiode 15 hindurchragt, welche unten durch Leitungen 17 mit dem Schaltwandler 8 verbunden ist. Das von dieser Leuchtdiode 15 abgestrahlte Licht wird dabei über Lichtleiter 18 durch den Hals 3 dem Kopf 2 zugeführt.

## Patentansprüche

1. Medizinisches Diagnostikgerät mit Beleuchtung durch mindestens eine, über eine oder mehrere Batterien (4) und einen Schaltwandler (8) gespeiste Leuchtdiode (12,15), aus einem, die Batterien (4) aufnehmenden Handgriff (1) oder dergleichen und einem auf diesem aufgesetzten Kopfteil (2) besteht, wobei der Schaltwandler (8) als Hülse in Form einer handelsüblichen Batterie gestaltet in den Handgriff (1) einsetzbar ist und die elektrischen Verbindungen mit der Batterie (4) und der oder den Leuchtdioden (12,15) über die Hülse (5) erfolgen, und wobei die Hülse (5) außen einen aus Metall bestehenden Mantel (6) aufweist, oben durch eine Kunststoffbuchse (9) abgeschlossen ist und unten einen Kunststoffsockel (7) aufnimmt, auf welchem sich der Schaltwandler (8) abstützt.

2. Medizinisches Diagnostikgerät nach Anspruch 1, **dadurch gekennzeichnet, dass** die Kunststoffbuchse mit einem Durchtritt für Stromleiter versehen ist.

3. Medizinisches Diagnostikgerät nach einem oder beiden der vorhergehenden Ansprüche, **dadurch gekennzeichnet, dass** die Hülse (5) in Form einer handelsüblichen Batterie gestaltet ist und anstelle einer zusätzlichen Batterie in den Handgriff (1) einsetzbar ist.

4. Medizinisches Diagnostikgerät nach einem oder mehreren der vorhergehenden Ansprüche, **dadurch gekennzeichnet, dass** die Hülse (5) an ihrem unteren Ende über eine Kontaktfläche mit dem einen Pol (13) und seitlich über eine innerhalb des Handgriffes (1) verlaufende Leitung (14) mit dem anderen Pol der Batterie (4) verbunden ist.

## Claims

1. Medical diagnostic device with illumination by at least one light-emitting diode (12, 15) powered by means of one or more batteries (4) and a switching converter (8), consisting of a handle (1) or the like which receives the batteries (4), and a head part (2) placed thereon, wherein the switching converter (8), constructed as a sleeve in the form of a commercially available battery, is insertable into the handle (1), and the electrical connections to the battery (4) and to the light-emitting diode(s) (12, 15) are made *via* the sleeve (5), and wherein the sleeve (5) has on the outside a jacket (6) consisting of metal, is closed at the top by a plastics bushing (9) and receives at the bottom a plastics base (7) on which the switching converter (8) is supported.

2. Medical diagnostic device according to claim 1, **characterised in that** the plastics bushing is provided with a passage for current conductors.

3. Medical diagnostic device according to one or both of the preceding claims, **characterised in that** the sleeve (5) is constructed in the form of a commercially available battery and is insertable into the handle (1) in place of an additional battery.

4. Medical diagnostic device according to one or more of the preceding claims, **characterised in that** the sleeve (5) is connected at its lower end to the one pole (13) of the battery (4) *via* a contact surface and is connected at the side to the other pole of the battery (4) *via* a line (14) extending inside the handle (1).

## Revendications

1. Appareil de diagnostic médical pourvu d'un éclairage grâce à au moins une DEL (12, 15) alimentée par le biais d'une ou de plusieurs batteries (4) et d'un convertisseur de tension de commutation (8), composé d'une poignée (1) recevant les batteries (4) ou similaires et une partie de tête (2) placée sur celle-ci, le convertisseur de tension de commutation (8) pouvant être inséré dans la poignée (1) comme une douille sous la forme d'une batterie usuelle disponible dans le commerce et les liaisons électriques avec la batterie (4) et la ou les DEL (12, 15) étant effectuées par le biais de la douille (5) et la douille (5) présentant à l'extérieur une enveloppe (6) en métal, étant terminée en haut par une bague en matière plastique (9) et recevant en bas un socle en matière plastique (7), sur lequel s'appuie le convertisseur de tension de commutation (8).

2. Appareil de diagnostic médical selon la revendication 1, **caractérisé en ce que** la bague en matière plastique est pourvue d'un passage pour les conducteurs.

3. Appareil de diagnostic médical selon l'une quelconque ou les deux revendications précédentes, **caractérisé en ce que** la douille (5) est réalisée sous la forme d'une batterie usuelle et peut être insérée dans la poignée (1) à la place d'une batterie supplémentaire.

4. Appareil de diagnostic médical selon l'une quelconque ou plusieurs revendications précédentes, **caractérisé en ce que** la douille (5) est reliée au niveau de son extrémité inférieure par le biais d'une surface de contact à un pôle (13) et latéralement par le biais d'un câble (14) s'étendant à l'intérieur de la poignée (1) à l'autre pôle de la batterie (4).
